# FASCICULE DE BREVET EUROPEEN

(11) **EP 2 580 044 B1**
(45) Date de publication et mention de la délivrance du brevet: **29.04.2020**
(21) Numéro de dépôt: 11725707.1
(22) Date de dépôt: 08.06.2011
(51) Int. Cl.: B29C 49/46, B29C 49/68, B29C 49/06, B29C 49/64, B65B 3/02, B65B 55/02, B67C 7/00, B67C 3/22, A61L 2/20

(54) **INSTALLATION DE FABRICATION DE RECIPIENTS COMPORTANT UN CIRCUIT DE RECYCLAGE DE L'AIR ET PROCEDE DE RECYCLAGE**
VORRICHTUNG ZUR HERSTELLUNG VON BEHÄLTERN MIT EINEM RECYCLING-KREISLAUF SOWIE RECYCLING-VERFAHREN
APPARATUS FOR MANUFACTURING CONTAINERS WITH A RECYCLING LOOP AND RECYCLING METHOD

(30) Priorité: 10.06.2010 FR 1054592
(43) Date de publication de la demande: 17.04.2013
(73) Titulaire: Sidel Participations, 76930 Octeville-sur-Mer (FR)
(72) Inventeur: ADRIANSENS, Eric, 76930 Octeville Sur Mer (FR)
(74) Mandataire: Grassin d'Alphonse, Emmanuel Jean Marie
(86) Numéro de dépôt international: PCT/EP2011/059492
(87) Numéro de publication internationale: WO 2011/154447

(56) Documents cités:
- EP-A1- 0 564 354
- EP-A1- 1 982 820
- EP-A1- 2 191 953
- JP-A- 2008 207 434
- US-A- 4 880 581
- US-A- 5 714 109

## Description

La présente invention concerne une installation de fabrication de récipients comportant un circuit de recyclage de l'air et un procédé de recyclage.

La présente invention concerne plus particulièrement une installation pour la fabrication de récipients à partir de préformes en matière thermoplastique, comportant au moins une enceinte de protection destinée à isoler un volume intérieur de l'installation par rapport à l'air ambiant compris dans un local industriel dans lequel est implantée l'installation, l'installation comportant agencées dans ledit volume intérieur au moins :
- une unité de conditionnement thermique des préformes constituée d'un four comportant des moyens de chauffage associés à un dispositif de refroidissement par air qui comporte des moyens de filtration de l'air ambiant puisé dans le local industriel par des moyens de ventilation pour assurer un refroidissement d'au moins une partie des préformes en transit à l'intérieur du four grâce audit air filtré ;
- une unité de transformation des préformes en récipients, et
- une unité de remplissage des récipients obtenus à partir desdites préformes.

On connaît des usines de production comportant au moins un bâtiment abritant au moins un local industriel et une telle installation de fabrication de récipients implantée dans ledit local.

Les installations de ce type sont destinées à la fabrication de récipients, notamment de bouteilles, de flacons, etc. à partir de préformes ou d'ébauches préalablement obtenues par injection de matière thermoplastique, en particulier de PET (PolyEthylène-Térephtalate).

De telles préformes sont préalablement conditionnées thermiquement dans un four formant l'unité de conditionnement thermique de l'installation afin de permettre leur transformation ultérieure en un récipient dans l'unité suivante, notamment par une opération de soufflage ou d'étirage-soufflage.

La maîtrise de l'étape de conditionnement thermique résulte de l'application conjuguée sur le corps de l'air de refroidissement et des rayonnements infrarouges émis par les moyens de chauffage pendant un laps de temps donné. Le refroidissement par air a pour effet de favoriser la chauffe par rayonnement en modérant les effets de conduction thermique au travers de la masse d'air contenue dans le four.

C'est la raison pour laquelle, le four d'une installation comporte un dispositif de refroidissement par air destiné à soumettre au moins le corps des préformes transitant au défilé à l'intérieur du four à un flux d'air de refroidissement.

Outre des moyens de ventilation destinés à établir ledit flux d'air de refroidissement à l'intérieur de la zone de chauffage du four, le dispositif de refroidissement comporte des moyens de filtration de l'air.

De tels moyens de filtration de l'air sont destinés à éliminer de l'air les impuretés (poussières, micro-organismes, ...) de manière que l'air de refroidissement délivré aux préformes soit un air filtré présentant un degré de propreté aussi élevé que possible.

Le four est encore équipé d'un système d'extraction d'air, tel qu'une hotte aspirante, qui comporte des moyens d'aspiration associés à un conduit d'évacuation pour extraire ledit air de refroidissement et évacuer cet air vers l'atmosphère.

Un tel système d'extraction permet alors d'évacuer avec l'air les calories résultant du chauffage opéré dans le four.

Dans certaines installations, un tel système d'extraction permet également d'évacuer des vapeurs résiduelles toxiques d'agent stérilisant lorsqu'une opération de décontamination de l'intérieur des préformes est mise en œuvre par application d'un agent stérilisant évaporé par chauffage dans le four, simultanément au conditionnement thermique.

Par conséquent, dans les différentes conceptions d'installation connues de l'état de la technique, l'air ambiant puisé dans le local industriel d'implantation et filtré par les moyens de filtration du dispositif de refroidissement équipant le four et ensuite évacué vers l'atmosphère, notamment l'intermédiaire d'un conduit d'évacuation d'un système d'extraction, c'est à dire rejeté à l'extérieur du local d'implantation de l'installation pour évacuer tant la chaleur que les résidus d'agent stérilisant.

Cependant, les consommations en air d'un tel dispositif de refroidissement dans les installations actuelles sont relativement importantes, les débits d'air sont par exemple de l'ordre de 20.000 à 30.000 m³ par heure selon le type de four.

Pour bien comprendre l'importance de la consommation en air ambiant d'un tel dispositif de refroidissement, on relèvera que le volume en air consommé par le dispositif de refroidissement en une heure de temps est généralement supérieur au volume du local industriel où le dispositif puise l'air ambiant.

Or comme "*la nature a horreur du vide*" (aphorisme d'Aristote), le volume d'air évacué vers l'atmosphère après refroidissement est instantanément remplacé dans le local industriel par un volume d'air équivalent.

Ainsi, l'air ambiant contenu dans le local industriel d'implantation est-il en permanence renouvelé et généralement par de l'air atmosphérique provenant de l'extérieur du bâtiment de l'usine de production abritant le local et son installation.

Cependant, l'air atmosphérique pénétrant depuis l'extérieur dans le local industriel présente des paramètres qualitatifs non maîtrisés.

En particulier, la température de l'air, son degré d'humidité ou encore la présence indésirable de bactéries comme de poussières ne sont pas maîtrisés et sont autant de paramètres dont il est en revanche parfaitement acquis qu'ils influencent directement la qualité de la fabrication des récipients.

De surcroît, de tels paramètres fluctuent en fonction de la localisation géographique du bâtiment comprenant l'installation, voir - pour une même localisation - en fonction de la saison qui est susceptible de modifier certains de ces paramètres qualitatifs comme la température de l'air (voir sur une même journée).

Les problèmes posés sont donc l'impact des variations des paramètres qualitatifs (température, humidité,...) de l'air atmosphérique renouvelant l'air ambiant du local industriel et tout particulièrement la propreté (poussières, bactéries, ...) de cet air conduisant à une dégradation de la qualité de fabrication des récipients, tout particulièrement la propreté des récipients.

De plus, cela a également pour conséquence un encrassement des moyens de filtration du dispositif de refroidissement se traduisant par une réduction de la durée de vie des moyens de filtration et une augmentation de la fréquence des opérations de maintenance, nécessitant pour l'un comme l'autre de procéder un arrêt complet de la fabrication.

L'incidence économique est donc directe sur les coûts d'exploitation de l'installation de fabrication de récipients.

Pour contrôler la qualité de l'air ambiant du local industriel, il est possible de recourir à une transformation du local industriel pour en faire une "salle blanche", c'est-à-dire une enceinte étanche aménagée pour éliminer le plus possible les poussières et les micro-organismes, pour obtenir des conditions industrielles de fabrication d'ultra propreté dans le local industriel.

Toutefois, on comprendra aisément que si une telle transformation est toujours possible, les coûts associés tant pour la transformation du local industriel qu'ultérieurement pour l'exploitation de l'installation sont très onéreux, prohibitifs.

Le but de la présente invention est notamment de remédier aux inconvénients précités et de proposer une installation particulièrement économique à l'exploitation, tout en étant aussi performante sur la qualité des récipients et avantageusement plus écologique.

Le document de brevet JP 2008 207434 A divulgue les caractéristiques du préambule de la revendication 1.

Dans ce but, l'invention propose une installation du type décrite précédemment, caractérisée en ce que l'installation comporte un circuit de recyclage de l'air de refroidissement comportant :
- des moyens d'extraction qui sont aptes à extraire hors du four l'air filtré introduit à l'intérieur du four par le dispositif de refroidissement par air, et
- au moins un conduit apte à amener au moins une partie dudit air filtré extrait du four jusqu'à au moins une unité de l'installation à l'intérieur de laquelle ledit air est introduit, grâce à quoi tout ou partie dudit air filtré initialement pour opérer un refroidissement par air dans le four est recyclé au moins directement dans l'unité de transformation et/ou l'unité de remplissage.

Avantageusement, un circuit de recyclage selon l'invention permet de recycler l'air filtré par le dispositif de refroidissement par air équipant le four, c'est-à-dire d'utiliser à nouveau directement "en boucle" l'air filtré après sa première utilisation dans le four aux fins de refroidissement.

Avantageusement, l'air de refroidissement recyclé présente des qualités particulièrement avantageuses par rapport à de l'air atmosphérique, lequel air atmosphérique aurait, dans une installation selon l'état de la technique, été admis dans le local industriel pour assurer le renouvellement de l'air auparavant intégralement évacué vers l'atmosphère.

En effet, l'air de refroidissement subit au moins une opération de filtration lors de son passage à travers les moyens de filtration du dispositif de refroidissement de sorte que cet air ensuite recyclé présente avant tout un degré de propreté particulièrement élevé, les poussières, bactéries, etc. en ayant été éliminées.

De plus, lors de son passage à travers le four, l'air de refroidissement s'assèche sous l'effet de la chaleur régnant à l'intérieur du four de sorte que l'on obtient une déshumidification de l'air sans avoir à recourir à des moyens supplémentaires.

Avantageusement, les paramètres de l'air ambiant du local sont ainsi maîtrisés, améliorant de facto la qualité générale des récipients produits par l'installation.

Avantageusement, le local industriel est pourvu de moyens de régulation de la température de l'air ambiant aptes à maintenir la température de l'air dans une plage de valeurs de température assurant des conditions de fabrication optimales.

Grâce au recyclage selon l'invention, on introduit dans l'unité de remplissage et/ou l'unité de transformation de l'air recyclé et cela en lieu et place d'air ambiant auparavant également aspiré dans le local industriel par un système d'insufflation équipant chacune de ces unités.

Avantageusement, la consommation totale en air de l'installation s'en trouve considérablement réduite dès lors que l'air de refroidissement recyclé depuis le four pourvoit directement au besoin de l'unité de remplissage et/ou de l'unité de transformation pour maintenir le volume intérieur d'au moins une unité en surpression afin d'éviter toute pollution des moyens de production s'y trouvant agencés.

Avantageusement, la consommation en air du dispositif de refroidissement du four est en effet supérieure à celle de l'unité de remplissage et de l'unité de transformation de sorte que le circuit de recyclage pourvoit intégralement à la totalité de l'air nécessaire pour la mise en surpression tant de l'unité de remplissage que de l'unité de transformation.

Avantageusement, l'air recyclé par le circuit de recyclage dans au moins une des unités de l'installation s'échappe ensuite à l'extérieur de l'enceinte pour retourner dans le local industriel d'implantation régénérant l'air ambiant aspiré par le dispositif de refroidissement.

L'air recyclé restitué à l'air ambiant est dès lors susceptible d'être à nouveau utilisé, en particulier aspiré une nouvelle fois par le dispositif de refroidissement et ainsi de suite suivant une circulation en boucle.

Du fait de la suppression de l'évacuation totale de l'air de refroidissement vers l'atmosphère, l'air ambiant n'est plus renouvelé par de l'air atmosphérique dès lors que le circuit de recyclage restitue l'air, avantageusement filtré, au local après son recyclage dans l'installation.

Grâce à un tel recyclage, l'air ambiant contenu dans le local industriel est un air dont la propreté croît avec la durée de fonctionnement de l'installation.

En effet, l'air ambiant puisé par le dispositif de refroidissement du four dans le local industriel est plus propre à chacun de ses passages à travers des moyens de filtration comportant au moins les moyens de filtration dudit dispositif et, de préférence, également les moyens de filtration respectifs de l'unité de remplissage et/ou de l'unité de transformation.

Avantageusement, le dispositif de refroidissement et les moyens du circuit de recyclage sont donc mis en fonctionnement pendant un laps de temps donné avant que ne débute réellement la fabrication des récipients et ceci afin d'améliorer la qualité de l'air ambiant contenu dans le local industriel.

Ainsi, lorsque l'on débute dans l'installation de fabrication le conditionnement thermique des préformes suivi de la transformation en récipients, l'air ambiant qui est puisé par le dispositif de refroidissement est un air beaucoup plus propre d'avoir été préalablement filtré pendant ledit laps de temps déterminé.

Avantageusement, la maîtrise de la qualité de l'air ambiant résultant de la mise en œuvre du recyclage selon l'invention permet d'augmenter la qualité de fabrication des récipients obtenus avec une telle installation, tout particulièrement le degré de propreté des récipients, critère de qualité notamment très contrôlé dans le cadre des applications agro-alimentaires.

De plus, l'encrassement de l'ensemble des moyens de filtration équipant l'installation s'en trouve réduit et leur durée de vie augmentée au bénéfice d'une réduction des coûts d'exploitation associés.

Avantageusement, le circuit de recyclage selon l'invention permet d'obtenir également des bénéfices économiques substantiels, notamment en raison de la réduction de la fréquence entre les opérations d'entretien ou de changements des moyens de filtration et de l'augmentation de la durée globale d'utilisation des moyens de filtration.

Selon d'autres caractéristiques de l'installation de fabrication selon l'invention :
- le circuit de recyclage comporte un conduit principal dont une extrémité amont est reliée aux moyens d'extraction et une extrémité aval reliée à l'unité de remplissage de manière que au moins une partie de l'air filtré extrait du four soit recyclée en étant introduite à l'intérieur de ladite unité de remplissage ;
- le circuit de recyclage comporte un conduit de dérivation dont une extrémité amont est reliée au conduit principal ou aux moyens d'extraction et dont l'autre extrémité aval est reliée à l'unité de transformation des préformes en récipients de manière que au moins une partie de l'air filtré extrait du four soit recyclée en étant introduite à l'intérieur de ladite unité de transformation ;
- l'unité de transformation et/ou l'unité de remplissage comportent des moyens de filtration pour filtrer l'air provenant du circuit de recyclage introduit dans l'une et/ou l'autre des unités de manière à réaliser une double filtration de l'air recyclé ;
- le circuit de recyclage comporte un conduit d'évacuation dont une extrémité amont communique avec les moyens d'extraction et dont l'autre extrémité aval débouche dans l'atmosphère, à l'extérieur du local industriel, et des moyens de régulation sont disposés de manière à répartir sélectivement l'air extrait dans ledit conduit d'évacuation et/ou dans ledit au moins un conduit du circuit de recyclage d'air ;
- l'installation comportant un module de décontamination des préformes par application d'au moins un agent stérilisant, ledit module de décontamination comporte des moyens d'extraction aptes à aspirer l'air comprenant de l'agent stérilisant à l'état gazeux hors d'une chambre de stérilisation du module de manière à évacuer hors du module ledit air chargé en agent stérilisant par l'intermédiaire d'au moins un conduit d'aspiration dont la sortie est reliée au conduit d'évacuation débouchant dans l'atmosphère ;
- le module de décontamination comporte des moyens d'extraction qui sont distincts des moyens d'extraction du circuit de recyclage destinés à aspirer l'air filtré hors du four ;
- le circuit de recyclage comporte des moyens de traitement aptes à neutraliser tout ou partie de l'agent stérilisant présent dans l'air recyclé ;
- les moyens de traitement de l'agent stérilisant sont agencés au moins dans le conduit apte à amener tout ou partie dudit air filtré extrait du four jusqu'à au moins une unité de l'installation ;
- des moyens de traitement de l'agent stérilisant sont agencés dans l'un et/ou l'autre desdits conduits d'aspiration et d'évacuation pour neutraliser tout ou partie dudit agent stérilisant présent dans l'air.

L'invention propose encore un procédé de recyclage de l'air dans une installation de fabrication de récipients à partir de préformes, comportant au moins les étapes consistant successivement à :
a) filtrer de l'air ambiant pour réaliser avec ledit air filtré un refroidissement par air d'au moins une partie des préformes au cours du conditionnement thermique opéré dans un four de l'installation ;
b) extraire l'air filtré hors du four après utilisation dudit air pour le refroidissement ;
c) recycler au moins une partie dudit air filtré extrait du four en introduisant tout ou partie dudit air dans une unité de transformation et/ou une unité de remplissage de l'installation.

Grâce au procédé de recyclage de l'air, on s'assure une maîtrise des paramètres qualitatifs de l'air ambiant et tout particulièrement on accroît le degré de propreté de l'air et donc incidemment celle des récipients fabriqués.

Avantageusement, les étapes du procédé de recyclage sont mises en œuvre dans une installation comportant un circuit de recyclage conforme aux enseignements de l'invention.

D'autres caractéristiques et avantages de l'invention apparaîtront à la lecture de la description détaillée qui va suivre pour la compréhension de laquelle on se reportera aux dessins annexés dans lesquels :
- la figure 1 est une vue schématique de dessus qui représente partiellement un exemple d'une installation de fabrication de récipients comportant un module de décontamination et illustrant l'état de la technique pour la demanderesse ;
- la figure 2 est une vue en perspective qui représente schématiquement une installation selon la figure 1 ;
- la figure 3 est une vue en perspective qui représente une installation de fabrication similaire à celle des figures 1 et 2 à l'exception de l'absence de module de décontamination et qui illustre un premier mode de réalisation d'un circuit de recyclage de l'air de refroidissement selon l'invention ;
- la figure 4 est une représentation de vues en coupe des unités de l'installation selon la figure 3, respectivement en coupe transversale pour le four et en coupe longitudinale pour les autres unités, et illustrant le circuit de recyclage selon le premier mode de réalisation ;
- la figure 5 est une vue en perspective qui représente schématiquement une installation de fabrication analogue à celui des figures 1 et 2 et comportant un module de décontamination des préformes et qui illustre un deuxième mode de réalisation d'un circuit de recyclage de l'air de refroidissement selon l'invention ;
- la figure 6 est une représentation de vues en coupe des unités de l'installation selon la figure 5, respectivement en coupe transversale pour le four et en coupe longitudinale pour les autres unités et du module de décontamination déporté dans un soucis de clarté de la représentation, l'ensemble illustrant le circuit de recyclage selon le deuxième mode de réalisation.

Dans la description et les revendications, on adoptera par convention et à titre non limitatif les termes "avant" ou "arrière" en référence à la direction longitudinale, "supérieur" et "inférieur", en référence à la direction verticale et selon la gravité terrestre, et les directions longitudinale, verticale et transversale en référence aux trièdres (L, V, T) indiqués sur les figures.

On utilisera les expressions "amont" et "aval" en référence au sens de circulation du flux d'air dans l'installation et/ou dans le circuit de recyclage de l'air.

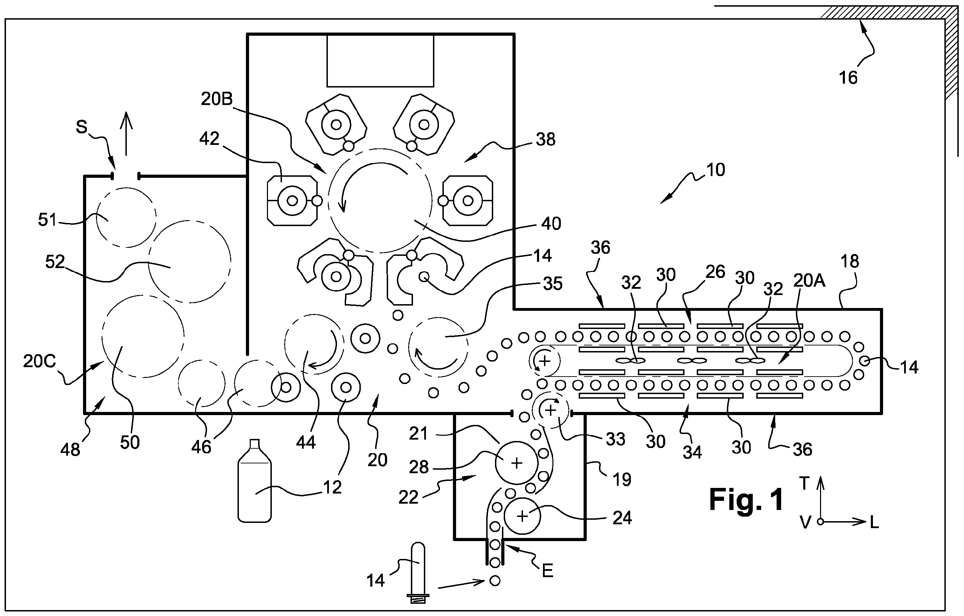

On a représenté schématiquement à la figure 1 une installation 10 pour la fabrication de récipients 12 à partir d'une préforme 14 en matière thermoplastique qui, de manière non limitative, illustre un exemple de réalisation d'une installation constituant l'état de la technique pour la demanderesse.

La figure 1 représente un exemple de préforme 14 pour la fabrication d'un récipient 12 formant un corps creux et notamment destinée à être conditionnée thermiquement puis transformée pour obtenir ledit récipient 12, tel qu'une bouteille, un flacon etc.

De préférence, l'opération de transformation de la préforme 14 conditionnée thermiquement est réalisée par soufflage ou par étirage-soufflage, en variante en tout ou partie par remplissage au moyen d'un liquide sous pression propre à former le récipient.

Par définition, le terme "récipient" désigne dans la suite de la présente description aussi bien le récipient final obtenu par un procédé de fabrication à une seule étape de transformation d'une préforme aboutissant directement au récipient final, telle que la bouteille illustrée en détail à la figure 1, qu'un récipient intermédiaire obtenu dans le cas de mise en œuvre d'un procédé à plusieurs étapes de transformation.

L'installation 10 est implantée dans un local industriel 16 généralement fermé se trouvant au sein d'un bâtiment (non représenté) d'une usine de production et qui présente un volume V d'air ambiant constituant un environnement extérieur déterminé autour de l'installation 10.

Avantageusement, l'installation 10 comporte au moins une enceinte 18 de protection de l'installation destinée à isoler, par rapport audit environnement extérieur formé par le local industriel 16 d'implantation de l'installation 10, un volume interne 20 à l'intérieur duquel sont agencés les différents moyens de fabrication des récipients 12.

L'installation 10 est associée à un système d'alimentation en préformes 14. Un tel système d'alimentation (non représenté) est par exemple décrit dans le document WO-2005/070793 auquel on reportera pour de plus amples détails.

Avantageusement, l'installation 10 représentée à la figure 1 comporte un module 22 de décontamination des préformes 14, notamment par application d'au moins un agent stérilisant.

En variante, l'installation 10 est toutefois susceptible d'être dépourvue d'un tel module 22 de décontamination. De préférence, le module 22 de décontamination est donc conçu comme un dispositif autonome afin de pouvoir être inclus sélectivement à l'installation 10 en fonction des applications.

A l'extrémité inférieure de rails du système d'alimentation, les préformes 14 sont saisies individuellement par une roue de chargement 24 agencée à l'entrée E de l'installation 10.

Avantageusement, le module 22 de décontamination comporte une enceinte 19 de protection qui est distincte de l'enceinte 18 globale délimitant le volume intérieur 20 comprenant lesdits moyens de fabrication de l'installation 10.

L'enceinte 19 de protection du module 22 de décontamination délimite également un volume intérieur formant une chambre 21 de stérilisation dans laquelle les préformes 14 sont traitées au moyen d'un agent stérilisant.

L'installation 10 comporte une unité de conditionnement thermique constituée principalement par au moins un four 26.

Avantageusement, le module 22 de décontamination est disposé en amont du four 26 de sorte que l'entrée E est aménagée dans l'enceinte 19 de protection du module 22 de décontamination. En variante, des moyens de décontamination sont agencés en aval, notamment après la sortie du four 26.

De préférence, le module 22 de décontamination est apte à projeter un jet de vapeur sèche sur les préformes 14 de manière à provoquer le dépôt par condensation d'un film de buée uniforme d'agent stérilisant sur au moins la paroi interne des préformes 14 à stériliser.

Avantageusement, l'agent stérilisant appliqué par le module 22 de décontamination à l'état de vapeur sèche comporte du peroxyde d'hydrogène (H₂O₂), par exemple un mélange d'eau et de peroxyde d'hydrogène qui est vaporisé grâce à des moyens de vaporisation par chauffage (non représentés) du module 22 de décontamination.

Le module 22 de décontamination comporte par exemple une roue 28 de stérilisation pourvue de moyens de support des préformes 14 grâce auxquels les préformes 14 circulent cols en haut sous des buses d'application du flux de vapeur sèche d'agent stérilisant pénétrant notamment dans chaque préforme 14 par l'ouverture délimitée par le col.

Un tel module 22 de décontamination est par exemple décrit dans le document FR-2.899.219 auquel on reportera pour une description détaillée dudit dispositif.

Les préformes 14 sont ensuite transférées de la roue 28 de stérilisation du module 22 de décontamination vers le four 26 grâce à une roue 33 de transfert et une ouverture aménagée dans l'enceinte 19 de protection.

L'unité de conditionnement thermique de l'installation 10 formée par le four 26 comporte principalement des moyens 30 de chauffage et des moyens 32 de ventilation associés pour procéder au conditionnement thermique des préformes 14.

Avantageusement, le four 26 comporte un dispositif 34 de refroidissement qui, associé aux moyens 30 de chauffage, comporte respectivement des moyens 36 de filtration et lesdits moyens 32 de ventilation.

En entrée du four 26, les préformes 14 sont transférées de la roue 33 à un dispositif de convoyage (non représenté) tel qu'une chaîne sans fin portant des moyens de support des préformes 14 et destiné à les transporter suivant un parcours de chauffe déterminé s'achevant à la sortie du four 26, par exemple ici un parcours en "U" comprenant deux tronçons longitudinaux de chauffage parallèles, l'un aller, l'autre retour, raccordés entre eux par un tronçon curviligne transversal, dit de stabilisation.

De préférence, les moyens de support du dispositif de convoyage sont aptes à entraîner en rotation sur elle-même chaque préforme 14 afin de favoriser une répartition appropriée de la chaleur dans le corps de chaque préforme 14.

Le document EP-1.699.613 décrit un exemple de four comportant des moyens 30 de chauffage et des moyens 32 de ventilation pour le conditionnement thermique de préformes.

Avantageusement, le four comporte des moyens de stérilisation par émission de rayonnement ultraviolet qui sont agencés au moins dans le tronçon curviligne de stabilisation tel que décrit dans le document WO-2008/049876.

Après leur conditionnement thermique par le four 26, les préformes 14 sont transportées par au moins une roue 35 de transfert de la sortie du four 26 jusqu'à une unité 38 de transformation des préformes 14 en récipients 12.

Avantageusement, l'unité 38 de transformation est ici constituée par une machine de soufflage, dite souffleuse.

Dans l'exemple représenté à la figure 1, la machine de soufflage est du type rotative et comporte un carrousel 40 équipé d'une pluralité de postes qui sont répartis circonférentiellement et qui sont chacun pourvus principalement de moyens 42 de moulage et de moyens de soufflage ou d'étirage-soufflage associés (non représentés).

De préférence, les récipients 12 obtenus sont ensuite transportés, par exemple par des roues 44, 46 de transfert, vers une unité 48 de remplissage.

Avantageusement, l'unité 48 de remplissage comporte au moins une machine 50 de remplissage et de préférence également une machine 52 de bouchage, lesdites machines 50, 52 (non représentées en détails) étant aptes à procéder successivement au remplissage des récipients 12 puis à la fermeture des récipients 12 remplis, par exemple au moyen de bouchons à vis complémentaires des cols.

De préférence, l'unité 48 de remplissage est juxtaposée à l'unité 38 de transformation de manière à obtenir une installation 10 compacte dans laquelle l'intégralité du processus de fabrication est mis en œuvre jusqu'à obtention finale de récipients 12 remplis et fermés, voir étiquetés.

Les récipients 12 sont par exemple acheminés vers une sortie S par une roue 51 de transfert et sont alors susceptibles d'être conditionnés, notamment mis en lots, emballés et palettisés, pour leur expédition aux fins de commercialisation.

Comme illustré sur la figure 1, l'enceinte 18 de l'installation 10 délimite un volume intérieur 20 général dans lequel sont disposés l'ensemble des moyens de fabrication 26, 38, 48 qui viennent d'être décrits et qui sont ainsi confinés, isolés de l'environnement extérieur.

Par convention, le volume intérieur 20 sera, dans la description qui suit, divisé en plusieurs volumes intérieurs 20A, 20B et 20C délimités par une partie correspondante de l'enceinte 18 et associés à chacune des unités, respectivement à l'unité de conditionnement thermique soit le four 26, à l'unité 38 de transformation et à l'unité 48 de remplissage.

L'enceinte 18 (matérialisée par un trait fort sur la figure 1) est par exemple constituée par un ensemble de parois verticales formées par des panneaux et d'au moins une paroi horizontale pour former un plafond recouvrant toute l'installation 10.

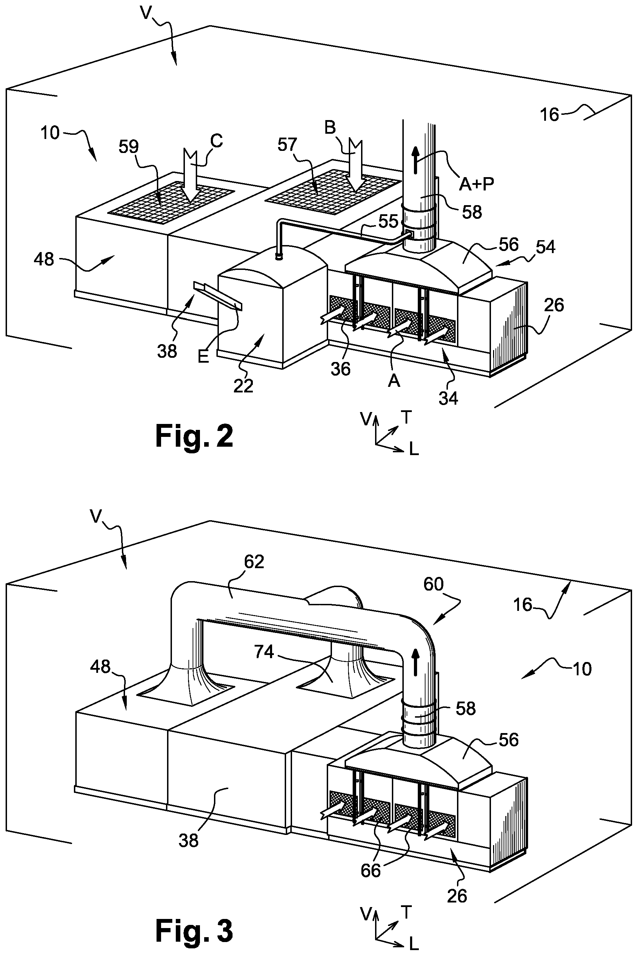

On décrira maintenant en référence à la figure 2, le fonctionnement et les circulations d'air se produisant entre l'air ambiant contenu dans le local industriel 16 et l'installation 10 qui s'y trouve implantée.

L'installation 10 comporte un système 54 d'extraction d'air qui, associé au four 26, comporte des moyens 56 d'extraction d'air destinés à extraire l'air de refroidissement hors dudit volume intérieur 20A délimité par la partie de l'enceinte 18 entourant le four 26 et ceci pour évacuer ledit air vers l'atmosphère tel que cela a été expliqué précédemment.

Dans une telle installation 10, la circulation de l'air dans le four 26 débute avec l'aspiration d'air ambiant qui est puisé dans le local industriel par les moyens 32 de ventilation du dispositif 34 de refroidissement.

De préférence, l'air est filtré par les moyens 36 de filtration lors de son introduction à l'intérieur du four 26.

Le flux d'air correspondant est illustré sur la figure 2 par des flèches "A" au voisinage des ouvertures d'admission de l'air destiné au refroidissement d'au moins une partie des préformes.

A l'intérieur du four 26, l'air filtré est amené jusqu'à la zone de chauffage des préformes 14 où il est vient en particulier au contact des corps des préformes 14 pour opérer leur refroidissement.

L'air de refroidissement est ensuite extrait depuis l'intérieur du four 26 par les moyens 56 d'extraction d'air du système 54 pour être intégralement rejeté par un conduit 58 d'évacuation vers l'atmosphère, soit à l'extérieur du bâtiment comportant le local industriel 16 d'implantation.

Lorsque l'installation 10 comporte un module 22 de décontamination, le système 54 d'extraction évacue également vers l'atmosphère l'air chargé en agent stérilisant extrait de la chambre 21 de stérilisation et qui est symbolisé par la lettre "P" sur la figure 2.

Pour ce faire, le module 22 de décontamination comporte un conduit 55 d'aspiration relié au conduit 58 d'évacuation débouchant dans l'atmosphère qui évacue alors l'air chargé en agent stérilisant issu du module 22 de décontamination et l'air de refroidissement du four 26.

Grâce à un tel système 54 d'extraction d'air, on évacue avec l'air, d'une part, la chaleur produite par le four 26 et, d'autre part, dans le cas d'une installation 10 comportant un module 22 de décontamination, l'agent stérilisant présent dans l'air à l'état gazeux.

Avantageusement, un système 57 d'insufflation d'air est associé à l'unité 38 de transformation comportant ici une machine de soufflage, ledit système 57 étant intégré dans une partie supérieure de l'enceinte 18 formant un plafond.

Ce système 57 d'insufflation est destiné à insuffler de l'air dans la partie associée de volume intérieur 20B, isolé de l'environnement extérieur, et ceci afin d'y établir une surpression permettant de réduire efficacement les risques de pollution depuis l'extérieur, notamment par des particules aéroportées.

Le système 57 d'insufflation puise donc dans le local industriel 16 un volume donné d'air ambiant qui est symbolisé par la flèche "B" sur la figure 2.

Un système 59 d'insufflation d'air analogue équipe l'unité 48 de remplissage afin d'établir également une surpression à l'intérieur du volume intérieur 20C délimité par l'enceinte 18, l'air ambiant puisé dans le local industriel 16 par le système 59 d'insufflation est symbolisé par une flèche "C" sur la figure 2.

A titre d'exemples non limitatifs, la consommation d'air ou débit d'air aspiré par un système 57 d'insufflation est de l'ordre de 8000 m³ par heure, tandis que celle du système 59 d'insufflation est de l'ordre de 10.000 m³ par heure.

Plus importante encore est l'air consommé par le dispositif 34 de refroidissement par air associé au four 26 puisque l'on atteint par exemple des valeurs de l'ordre de 20.000 à 30.000 m³ par heure selon le type de four, en particulier le nombre de module de chauffe.

Pour un four 26 de conception modulaire, la consommation d'air par module de chauffage est de l'ordre de 1300 m³ par heure, le four pouvant comporter par exemple 20 modules.

Comme expliqué précédemment, la quantité d'air ambiant puisée dans le local industriel 16 par le dispositif 34 de refroidissement et son évacuation ensuite vers l'atmosphère provoquent une admission massive d'air atmosphérique dans le local industriel 16 pour compenser l'air de refroidissement qui est évacué par le conduit 58 d'évacuation du système 54 d'extraction.

Or, le renouvellement de l'air ambiant par de l'air atmosphérique conduit à une importante variation des paramètres qualitatifs de l'air (température, humidité, propreté...) qui affectent directement le procédé de fabrication et tout particulièrement le conditionnement thermique.

Surtout, l'absence de propreté (poussières, micro-organismes, ...) de l'air atmosphérique conduit à dégrader la qualité des récipients 12 fabriqués.

De plus, cela conduit également à un encrassement prématuré des moyens 36 de filtration du dispositif 34 de refroidissement.

Une des conséquences est la réduction de la durée de vie des moyens de filtration et l'augmentation de la fréquence des opérations de maintenance nécessitant un arrêt complet de la fabrication, faute de quoi le degré de propreté des récipients 12 fabriqués s'en trouve directement affecté et rapidement dégradé.

L'incidence négative concerne donc autant les coûts d'exploitation de l'installation 10 de fabrication de récipients que le degré de propreté des récipients 12 fabriqués.

Dans le but de résoudre notamment ces inconvénients, l'invention propose de recycler l'air de refroidissement extrait du four afin de supprimer le renouvellement de l'air ambiant par de l'air atmosphérique, tout en tirant avantageusement profit du fait que cet air est un air filtré pour améliorer la qualité des récipients fabriqués et tout particulièrement le degré de propreté.

Pour ce faire, l'invention propose un procédé de recyclage de l'air dans une installation 10 de fabrication de récipients 12 à partir de préformes 14, comportant au moins les étapes consistant successivement à :
a) filtrer de l'air ambiant pour réaliser avec ledit air filtré un refroidissement par air d'au moins une partie des préformes 14 au cours du conditionnement thermique opéré dans un four 26 de l'installation 10 ;
b) extraire l'air filtré hors du four 26 après utilisation dudit air pour le refroidissement ;
c) recycler au moins une partie dudit air filtré extrait du four 26 en introduisant tout ou partie dudit air dans une unité 38 de transformation et/ou une unité 48 de remplissage de l'installation 10.

Conformément au procédé de recyclage selon l'invention, l'air filtré par le dispositif de refroidissement est recyclé, c'est-à-dire préférentiellement introduit directement dans au moins l'une des unités 38, 48 de l'installation 10 de manière à pouvoir être utilisé à nouveau en tant qu'air ambiant.

Avantageusement, dans le cas d'une évacuation directe dans au moins une unité 38, 48, le recyclage de l'air de refroidissement permet de mettre fin au phénomène de renouvellement par de l'air atmosphérique survenant jusqu'alors dans les installations dans lesquelles cet air était évacué vers l'atmosphère.

En effet, l'air recyclé par le circuit de recyclage dans au moins une des unités de l'installation s'échappe ensuite à l'extérieur de l'enceinte pour retourner dans le local industriel d'implantation où il peut d'être à nouveau utilisé par le dispositif 34 de refroidissement.

Grâce à l'invention, l'air ambiant contenu dans le local industriel 16 est un air dont la propreté va croître avec la durée de fonctionnement de l'installation 10, les poussières, bactéries, etc. étant éliminées par les opérations de filtration successives.

Avantageusement, la maîtrise de la qualité de l'air ambiant résultant de la mise en œuvre du recyclage selon l'invention permet d'augmenter la qualité de fabrication des récipients obtenus avec une telle installation, tout particulièrement le degré de propreté.

On décrira maintenant à titre d'exemples non limitatifs, un premier mode de réalisation et un deuxième mode de réalisation préférés de l'invention qui illustrent respectivement la mise en œuvre du procédé de recyclage dans une installation 10 de fabrication de récipients 12.

Conformément à l'invention, l'installation 10 comporte un circuit 60 de recyclage de l'air de refroidissement comportant :
- des moyens 56 d'extraction qui sont aptes à extraire l'air filtré introduit à l'intérieur du four 26 par le dispositif 34 de refroidissement par air, et
- au moins un conduit apte à amener au moins une partie dudit air filtré extrait du four 26 jusqu'à au moins une unité 38, 48 de l'installation à l'intérieur de laquelle ledit air est introduit, grâce à quoi tout ou partie dudit air filtré initialement pour opérer un refroidissement par air dans le four 26 est recyclé au moins dans l'unité 38 de transformation et/ou l'unité 48 de remplissage.

Avantageusement, le circuit 60 de recyclage comporte au moins un conduit 62, 74 dont une extrémité amont est reliée à des moyens 56 d'extraction et dont l'autre extrémité aval est reliée à l'une des unités 38, 48.

Grâce au circuit 60 de recyclage selon l'invention, l'air ambiant initialement filtré par les moyens 36 de filtration pour assurer le refroidissement dans le four 26 est recyclé dans au moins une unité de l'installation 10, telle que l'unité 38 de transformation et/ou l'unité 48 de remplissage.

Avantageusement, l'air filtré pour le refroidissement n'est donc plus rejeté dans l'atmosphère mais utilisé à nouveau pour l'installation 10.

Le recyclage selon l'invention a pour premier effet la suppression de l'appel d'air qui conduisait auparavant à ce que de l'air atmosphérique pénètre dans le local industriel 16 en compensation de l'air évacué dans l'atmosphère.

Avantageusement, on limite les variations des paramètres qualitatifs de l'air dans le local industriel 16, en particulier la propreté.

En effet, l'air recyclé est indirectement restitué au local industriel 16 après avoir traversé par exemple l'unité 48 de remplissage dont le volume intérieur 20C est maintenu en surpression à l'intérieur de l'enceinte 18.

L'air filtré étant recyclé dans au moins une unité où il est introduit par le conduit du circuit 60 de recyclage, un deuxième effet est qu'il n'est dès lors plus nécessaire de puiser, dans l'air ambiant du local industriel 16, l'air auparavant nécessaire à la mise en surpression de ladite au moins une unité 38, 48, c'est-à-dire en référence à la figure 2 aux flux d'air correspondant à la flèche B et/ou à la flèche C.

En effet, l'air nécessaire à la mise en surpression du volume intérieur 20B de l'unité 38 de transformation et/ou du volume intérieur 20C de l'unité 48 de remplissage est avantageusement intégralement constitué par de l'air recyclé délivré par le circuit 60 de recyclage de l'air.

Avantageusement, l'air recyclé ayant été filtré par les moyens 36 de filtration, l'air délivré par le circuit 60 est un air présentant un degré de propreté qui, déterminé par les moyens de filtration employés, est supérieur à celui initial de l'air ambiant.

De préférence, les moyens 36 de filtration du dispositif 34 de refroidissement sont constitués par des filtres dits "absolus", tels que des filtres de type « ULPA », voir de type « HEPA ».

On rappellera qu'un filtre « ULPA » acronyme pour « *Ultra Low Penetration Air »* en anglais est appelé en français : filtre à air à très faible pénétration, et qu'un filtre « HEPA » acronyme pour « *High Efficiency Particulate Air* » en anglais est lui appelé en français : filtre à air à très haute efficacité.

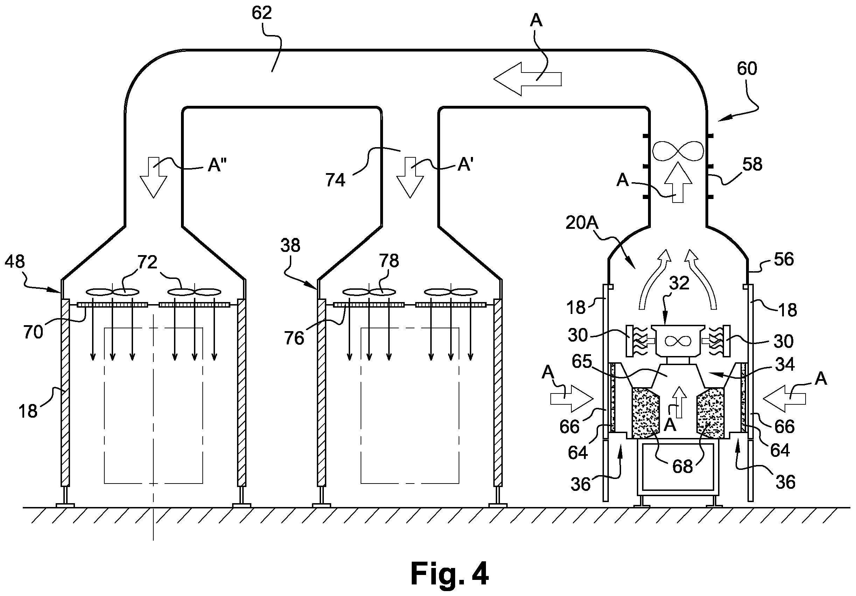

De préférence et tel qu'illustré par la coupe transversale du four 26 à la figure 4, les moyens 36 de filtration comportent un pré-filtre 64 qui est agencé au voisinage d'un bouche 66 d'admission disposée dans l'enceinte et un filtre 68 qui est agencé au voisinage d'un orifice aval de la bouche 64 d'admission, la section de passage du pré-filtre 66 étant supérieure à la section de passage du filtre 68.

De préférence, le pré-filtre 64 est un filtre gravimétrique, qui est apte à filtrer des particules dites grossières, qui sont d'un diamètre déterminé, par exemple supérieur à dix microns.

Avantageusement, le filtre 68 est un filtre « absolu » d'un des types précités, qui est apte à filtrer des particules dites fines, par exemple d'un diamètre supérieur à un micron.

Dans le premier mode de réalisation de l'invention représenté aux figures 3 et 4 qui sera décrit ci-après par comparaison, l'installation 10 diffère de celle décrite précédemment en référence aux figures 1 et 2 en ce qu'elle ne comporte pas de module 22 de décontamination.

Par conséquent, l'air filtré qui est recyclé grâce au circuit 60 de recyclage selon le premier mode de réalisation est un air totalement exempt d'agent stérilisant.

Dans ce premier mode de réalisation, le circuit 60 de recyclage permet avantageusement de recycler l'air vers l'unité 48 de remplissage et vers l'unité 38 de transformation.

En variante, le circuit 60 de recyclage permet de recycler l'air vers une seule unité, soit vers l'unité 48 de remplissage ou vers l'unité 38 de transformation

Le circuit 60 de recyclage comporte un conduit 62, dit principal, de manière qu'au moins une partie de l'air filtré extrait du four 26 soit recyclée en étant introduite à l'intérieur de ladite unité 48 de remplissage.

De préférence, le conduit principal 62 comporte une extrémité amont reliée aux moyens 56 d'extraction et une autre extrémité aval reliée à l'unité 48 de remplissage.

Le circuit 60 de recyclage comporte un conduit 74, dit de dérivation, de manière qu'au moins une partie de l'air filtré extrait du four 26 soit recyclée en étant introduite à l'intérieur de ladite unité 38 de transformation.

De préférence, le conduit 74 de dérivation comporte une extrémité amont reliée au conduit principal 62 et une autre extrémité aval reliée à l'unité 38 de transformation des préformes 14 en récipients 12.

En variante, le conduit 74 de dérivation comporte une extrémité amont reliée aux moyens 56 d'extraction et une autre extrémité aval reliée à l'unité 38 de transformation des préformes 14 en récipients 12.

Selon cette variante, le conduit 74 est alors le seul conduit du circuit 60 de sorte que le recyclage s'effectue uniquement vers l'unité 38 de transformation des préformes 14 en récipients 12.

De préférence, l'unité 48 de remplissage comporte des moyens 70 de filtration qui sont interposés entre une extrémité aval du conduit principal 62 et le volume intérieur 20C de l'unité 48 de remplissage délimité par une partie de l'enceinte 18 de protection.

Avantageusement, l'air filtré introduit dans l'unité 48 de remplissage est un air doublement filtré dès lors que l'air est filtré une première fois par les moyens 36 de filtration du dispositif 34 de refroidissement puis une seconde fois par les moyens 70 de filtration associés à l'unité 48 de remplissage.

De préférence, l'unité 48 de remplissage comporte des moyens 72 de ventilation aptes à créer une dépression et un appel de l'air filtré en circulation dans le conduit principal 62 de manière à compenser en partie les pertes de charge et à garantir un débit d'air propre à maintenir le volume intérieur 20C en surpression.

Avantageusement, une partie de l'air recyclé circulant dans le conduit principal 62 est dérivée vers le conduit 74 de dérivation, relié ici au conduit principal 62, de manière à dériver une partie de l'air vers l'unité 38 de transformation de sorte que l'air recyclé se réparti entre l'unité 48 de remplissage et l'unité 38 de transformation.

On établit alors une surpression dans le volume intérieur 20B de l'unité 38 de remplissage.

Avantageusement et tel qu'illustré sur la figure 4, l'unité 38 de transformation comporte des moyens 76 de filtration et des moyens 78 de ventilation analogues à ceux de l'unité 48 de remplissage.

Grâce à de tels moyens 76 de filtration, on assure également une double filtration de l'air recyclé introduit dans l'unité 38 de transformation qui sera en contact avec les préformes 14 ou les récipients 12 de sorte que ces derniers sont maintenus dans un environnement contrôlé permettant de maintenir un degré de propreté très élevé.

Avantageusement, les moyens 78 de ventilation permettent de garantir l'établissement de la surpression dans le volume intérieur 20B et de compenser toute perte de charge éventuelle dans le circuit 60 de recyclage.

Avantageusement, l'unité 38 de transformation et/ou l'unité 48 de remplissage comportent des moyens 76, 70 de filtration pour filtrer l'air provenant du circuit 60 de recyclage introduit dans l'une et/ou l'autre des unités 38, 48 de manière à réaliser une double filtration de l'air recyclé.

En variante, l'unité 38 de transformation et/ou l'unité 48 de remplissage sont dépourvues de tels moyens de filtration et de ventilation et l'air recyclé introduit directement dans le volume intérieur 20B et/ou 20C.

Avantageusement, le circuit 60 de recyclage selon l'invention permet donc d'alimenter en air tant l'unité 48 de remplissage que l'unité 38 de transformation, pour établir respectivement la mise en suppression des volumes intérieurs 20B, 20C de chacune desdites unités 38, 48.

En variante non représentée, le circuit 60 de recyclage alimente en air uniquement une seule unité, par exemple l'unité 48 de remplissage.

L'unité 38 de transformation n'est alors pas raccordée et le conduit 74 de dérivation supprimé. Avantageusement, ladite unité 38 de transformation comporte alors des moyens 57 d'insufflation d'air aptes à établir une surpression dans le volume intérieur 20C.

Bien entendu et réciproquement, le circuit 60 de recyclage pourrait n'alimenter que l'unité 38 de transformation grâce au conduit 74, le conduit principal 62 étant alors supprimé mais des moyens 59 d'insufflation étant mis en œuvre.

Grâce à l'invention, lorsque l'air est recyclé vers les deux unités 38 et 48, il n'est dès lors plus nécessaire de puiser dans l'air ambiant du local industriel 16 ce qui participe encore à la suppression de l'introduction d'air atmosphérique dans le local industriel 16.

En effet, seul le dispositif 34 de refroidissement puise alors l'air ambiant contenu dans le local industriel 16, ledit air ambiant puisé étant compensé dans le local industriel 16 par l'air recyclé restitué de manière continue par chacune des unités 38 de transformation et 48 de remplissage en surpression.

Avantageusement, l'air ambiant puisé par le dispositif 34 de refroidissement est successivement filtré par deux fois, respectivement dans le dispositif 34 de refroidissement puis lors du recyclage par les unités 38, 48, de sorte que le degré de propreté de l'air restitué finalement au local industriel 16 après une seule "boucle" est déjà très supérieur à celui initial.

En effet, une fois restitué au local industriel 16, cet air forme une partie de l'air ambiant et se trouve alors susceptible d'être de nouveau aspiré par le dispositif 34 de refroidissement.

On comprendra dès lors qu'à chaque passage ou boucle à travers le circuit 60 de recyclage de l'installation 10, l'air restitué en sortie est un air de plus en plus propre.

C'est la raison pour laquelle, le recyclage de l'air réalisé conformément aux enseignements de l'invention permet d'améliorer la qualité des récipients 12 fabriqués qui présentent ainsi un degré de décontamination ou stérilité particulièrement élevé.

Avantageusement, le recyclage de l'air de refroidissement selon l'invention permet de réduire l'encrassement des moyens 36 de filtration comme des moyens 70, 76 de filtration des unités 48, 38, et de réduire la fréquence des interventions de maintenance.

La qualité de l'air ambiant est substantiellement accrue dès lors que l'air est filtré par les moyens de filtration ou encore automatiquement déshumidifié par les moyens 30 de chauffage du four 26.

On décrira maintenant, en référence à la figure 4, le fonctionnement d'un circuit 60 de recyclage selon le premier mode de réalisation de l'invention.

L'air ambiant est puisé dans le local industriel 16 par le dispositif 34 de refroidissement associé au four 26 et destiné au refroidissement par air d'au moins une partie des préformes 14.

Plus précisément, les moyens 32 de ventilation aspirent l'air ambiant par les bouches 66 d'admission en provoquant une dépression, de préférence dans un plénum 65. L'air ambiant ainsi aspiré va alors être filtré par les moyens 32 de filtration du dispositif 34, par exemple successivement filtré par un pré-filtre 64 puis par un filtre 66 disposé en aval du pré-filtre 64.

Depuis le plénum 65 formant un réservoir d'air de refroidissement en partie inférieure du four 26, l'air filtré est alors aspiré par les moyens 32 de ventilation agencés dans la zone de chauffage de manière à souffler l'air de refroidissement, généralement à travers des réflecteurs, dans ladite zone comportant les moyens 30 de chauffage des préformes 14.

Les moyens 32 de ventilation sont par exemple constitués par une roue à écopes apte à propulser l'air de refroidissement dans la zone de chauffage au moins vers les corps des préformes 14 en cours de conditionnement thermique.

Après quoi l'air de refroidissement est extrait par le haut par les moyens 56 d'extraction du circuit 60 de recyclage de l'air selon l'invention.

Tel que représenté sur la figure 4, les flèches A illustrent successivement le parcours de l'air de refroidissement jusqu'à son extraction par les moyens 56 d'extraction disposés au dessus du four 26, les moyens 56 d'extraction fermant par le haut le volume intérieur 20A délimité par une partie de l'enceinte 18.

L'air filtré ainsi extrait est alors recyclé par l'intermédiaire du circuit 60 qui achemine au moins une partie de l'air.

D'une part, pour une première partie représentée par la flèche A', l'air est acheminé jusqu'à l'unité 38 de transformation par l'intermédiaire du conduit principal 62 et du conduit 74 de dérivation et, d'autre part, pour l'autre partie correspondant à la flèche A" acheminé jusqu'à l'unité 48 de remplissage par l'intermédiaire du conduit principal 62.

En variante, l'intégralité du flux d'air recyclé représenté par la flèche A est introduit dans l'unité 48 de remplissage ou dans l'unité 38 de transformation.

La première partie de cet air parvenu à l'unité 38 de transformation suivant la flèche A' est alors filtré une deuxième fois par les moyens 76 de filtration équipant l'unité 38 et introduit dans le volume intérieur 20B associé afin de maintenir en permanence une surpression dans cette partie correspondante de l'enceinte 18.

Avantageusement, les moyens 78 de ventilation provoquent une aspiration dans le conduit 74 de dérivation afin de maintenir un débit de circulation d'air élevé et la surpression dans l'unité 38 de transformation.

La deuxième partie de l'air parvenu à l'unité 48 de remplissage suivant la flèche A", avantageusement avec l'assistance des moyens 72 de ventilation, est alors filtré une deuxième fois par les moyens 70 de filtration équipant l'unité 48 et introduit dans le volume intérieur 20C associé afin de maintenir en permanence une surpression dans l'enceinte 18 autour des machines 50 et 52.

L'air s'échappe ensuite de l'unité 38 de transformation comme de l'unité 48 de remplissage par la partie inférieure de l'enceinte 18, ici au voisinage du sol, de manière que cet air est alors restitué au local industriel 16.

Conformément aux enseignements de l'invention, l'air ainsi recyclé prévient la pénétration dans le local industriel 16 d'air atmosphérique et permet d'assurer un air ambiant de grande qualité, présentant en particulier un degré élevé de propreté.

En effet, grâce au recyclage, le renouvellement de l'air ambiant par de l'air atmosphérique est supprimé de sorte que les inconvénients précités sont également supprimés.

Afin de bien comprendre les avantages de l'invention, on détaillera ci-après les consommations d'air ou débits d'une installation 10 de fabrication du type de celle décrite en référence aux figures 1 et 2.

La consommation globale en air d'une installation 10 est de l'ordre de 30.000 à 50.000 m³ par heure avec globalement la répartition suivante : environ 20.000 à 30.000 m³ pour le dispositif 34 de refroidissement par air du four et 8000 à 12.000 m³ pour le système 57 d'insufflation de l'unité 38 de transformation, comme pour le système 59 d'insufflation de l'unité 48 de remplissage qui est généralement d'environ 8000 m³ par heure.

Pour illustrer l'importance de telles consommations en air, il est utile d'établir une comparaison avec le volume global du local industriel 16 d'implantation de l'installation 10.

Ainsi à titre d'exemple non limitatif, si l'on considère un local industriel 16 globalement parallélépipédique présentant des dimensions respectivement de 6 m pour la hauteur, de 15 m de largeur et 30 m de longueur, on obtient alors un volume de 2700 m³.

Le volume de 2700 m³ d'air ambiant du local industriel 16 constitue un "réservoir" dans lequel l'air nécessaire à l'installation 10 est puisé et ceci tant par le dispositif 34 de refroidissement que par les moyens d'insufflation 57, 59 pour la mise en surpression des unités 38, 48.

En considérant une consommation en air égale à 27.000 m³ par heure pour le seul dispositif 34 de refroidissement dont l'air est évacué dans l'atmosphère, cela signifie que l'air ambiant contenu dans ce volume du local industriel 16 va être renouvelé plus de 10 fois en une heure de temps avec de l'air provenant de l'atmosphère pour compenser celui évacué.

On comprendra dès lors aisément combien les problèmes des paramètres qualitatifs associés au renouvellement de l'air par de l'air atmosphérique sont d'importance et impactent très significativement la qualité de fabrication des récipients, en perturbant notamment le conditionnement thermique dans le four 26.

Dans une installation 10 comportant un circuit 60 de recyclage selon l'invention, en supprimant l'évacuation de l'air de refroidissement vers l'atmosphère, on va renouveler avec de l'air recyclé de plus en plus propre le volume V du local industriel 16.

Avantageusement, l'air recyclé est directement réutilisé dans l'unité 48 de remplissage et dans l'unité 38 de transformation de l'installation 10, de préférence en établissant de surcroît une double filtration.

En variante non représentée, le circuit 60 de recyclage selon le premier mode de réalisation comporte un conduit d'évacuation pour dériver sélectivement au moins une partie de l'air extrait et ne réaliser qu'un recyclage partiel vers au moins une unité de l'installation.

De préférence, un tel conduit d'évacuation est analogue au conduit 58 de la figure 2 et comporte une extrémité amont qui communique avec les moyens 56 d'extraction et une autre extrémité aval qui débouche dans l'atmosphère, à l'extérieur du local industriel 16.

Avantageusement, des moyens de régulation du flux sont alors disposés de manière à commander sélectivement la répartition de l'air extrait dans le conduit principal 62 et/ou dans ledit conduit d'évacuation.

On décrira maintenant par comparaison, avec le premier mode de réalisation, un deuxième mode de réalisation de l'invention illustrant respectivement la mise en œuvre du procédé de recyclage selon l'invention dans une installation 10 de fabrication de récipients 12 comportant un module 22 de décontamination.

Le module 22 de décontamination est analogue à celui décrit précédemment en référence aux figures 1 et 2, notamment en ce qu'il est avantageusement apte à décontaminer les préformes 14 en déposant par condensation de l'agent stérilisant projeté sous la forme d'un flux de vapeur sèche.

Avantageusement, le module 22 de décontamination comporte des moyens 53 d'extraction aptes à aspirer l'air comprenant l'agent stérilisant à l'état gazeux hors de la chambre 21 de stérilisation du module 22 de décontamination.

Le module 22 de décontamination comporte au moins un conduit 55 d'aspiration de manière à évacuer hors de la chambre 21 de stérilisation ledit air chargé en agent stérilisant aspiré grâce aux moyens 53 d'extraction.

De préférence, les moyens 53 d'extraction sont distincts des moyens 56 d'extraction associés au four 26.

Comme pour le premier mode de réalisation, le circuit 60 de recyclage de l'air de refroidissement comporte des moyens 56 d'extraction, tels qu'une hotte, pour extraire hors du volume intérieur 20A du four 26 l'air de refroidissement préalablement filtré par les moyens 36 de filtration du dispositif 34 de refroidissement et comporte un conduit principal 62 apte à acheminer l'air extrait pour le recycler.

Dans ce deuxième mode de réalisation, l'air est de préférence uniquement recyclé vers l'unité 48 de remplissage et ceci en raison de la présence d'agent stérilisant dans cet air.

En effet, on rappellera que l'agent stérilisant est évaporé dans le four 26 par les moyens 30 de chauffage de sorte que l'air extrait comporte de l'agent stérilisant à l'état gazeux, bien qu'en relativement faible concentration.

Toutefois, la quantité d'agent stérilisant employée avec un module 22 de décontamination à dépôt par condensation est avantageusement réduite par comparaison aux techniques antérieures par mouillage ou pulvérisation d'une solution liquide.

En raison de la présence d'agent stérilisant dans l'air recyclé et du caractère généralement corrosif de ce type d'agent, comme le peroxyde d'hydrogène, l'air n'est pas dans l'unité 38 de transformation.

En variante, l'air recyclé pourrait cependant être introduit dans l'unité 38 de transformation en utilisant des moyens de traitement appropriés aptes à neutraliser totalement l'agent stérilisant présent dans l'air avant son introduction dans l'unité 38 de transformation.

Avantageusement, l'air recyclé par le circuit 60 présente un degré de stérilité encore accru du fait que, outre la ou les filtrations subies, l'agent stérilisant présent dans l'air exerce un effet stérilisant en raison notamment de ses propriétés bactéricides.

En revanche, la présence d'agent stérilisant dans l'air recyclé vers l'unité 48 de remplissage est avantageuse.

En effet, l'unité 48 de remplissage est usuellement considérée comme l'unité de l'installation 10 sur laquelle la plus grande attention est portée quant aux risques de pollution ou de contamination afin de garantir la qualité finale des récipients 12 fabriqués.

Par ailleurs, les machines 50 de remplissage ou 52 de bouchage de l'unité 48 de remplissage sont généralement réalisées avec des matériaux inoxydables, comme l'inox, pour permettre des traitements de décontamination de l'unité 48 à l'aide également d'agent stérilisant.

C'est la raison pour laquelle, la présence d'agent stérilisant dans l'air recyclé est sans risque de corrosion pour ces machines 50, 52 et autres moyens compris dans l'unité 48 de remplissage.

Avantageusement, l'air recyclé chargé en agent stérilisant va permettre de stériliser les moyens 70 de filtration de l'unité 48 de remplissage lors de son passage à travers ces derniers grâce à quoi on obtient une action stérilisante sur lesdits moyens 70 de filtration, la stérilité d'origine des moyens 70 de filtration étant prolongée par la présence d'agent stérilisant dans l'air recyclé.

Par conséquent, on améliore encore ainsi la propreté des récipients 12 fabriqués.

De préférence, des moyens de traitement aptes à neutraliser l'agent stérilisant présent dans l'air recyclé sont intégrés au circuit 60 de recyclage de manière à garantir que la concentration en agent stérilisant dans l'air recyclé reste inférieure à une valeur de seuil déterminée.

De tels moyens de traitement sont par exemple agencés en amont des moyens 70 de filtration, en variante dans le conduit principal 62.

Avantageusement, les moyens de traitement du circuit 60 de recyclage comportent au moins un élément oxydant apte à provoquer une réaction catalytique au cours de laquelle tout ou partie de l'agent stérilisant présent dans l'air extrait est décomposé en d'autres produits neutres.

De préférence, l'élément oxydant est constitué par un composé comportant du platine, de l'argent ou tout autre métal approprié pour réaliser la catalyse.

Lorsque l'agent stérilisant utilisé est avantageusement du peroxyde d'hydrogène, on décompose alors celui-ci en eau et en dioxygène, soit des produits ne présentant aucune nocivité.

De préférence, les moyens de traitement sont constitués par au moins un filtre comportant au moins ledit élément oxydant destiné à interagir avec l'agent stérilisant présent dans l'air extrait, par exemple un filtre pourvu d'un substrat actif comportant ledit élément oxydant.

De préférence, le circuit 60 de recyclage comporte un conduit 58 d'évacuation, analogue à celui de la figure 2, et comporte une extrémité amont qui communique avec les moyens 56 d'extraction et une autre extrémité aval qui débouche dans l'atmosphère, à l'extérieur du local industriel 16 notamment pour évacuer une partie de l'air de refroidissement, sans en recycler la totalité.

Avantageusement, des moyens 80 de régulation du flux sont disposés de manière à commander sélectivement la répartition de l'air extrait dans le conduit principal 62 et/ou dans ledit conduit 58 d'évacuation.

A titre d'exemple, les moyens 80 de régulation sont constitués par un volet monté mobile entre au moins une première position extrême dans laquelle le volet s'étend horizontalement pour obturer l'entrée du conduit 58 d'évacuation et une deuxième position extrême dans laquelle le volet s'étend verticalement pour obturer l'entrée du conduit principal 62 du circuit 60 de recyclage.

Avantageusement, le volet est susceptible d'occuper une ou des positions intermédiaires entre lesdites positions extrêmes de manière à répartir l'air filtré extrait entre les conduits 58, 62.

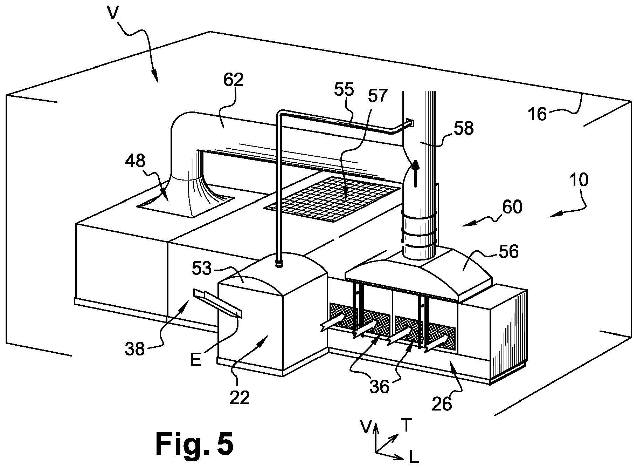

De préférence et tel qu'illustré sur la figure 5, la sortie du conduit 55 d'aspiration du module 22 de décontamination est reliée au conduit 58 d'évacuation du circuit 60 de recyclage de manière que l'air chargé en agent stérilisant extrait par les moyens 53 d'extraction de la chambre 21 de stérilisation soit évacué directement vers l'atmosphère.

Avantageusement, le raccordement de la sortie du conduit 55 d'aspiration sur le conduit 58 d'évacuation est réalisé en aval du conduit principal 62 et des moyens 80 de régulation.

A titre d'exemple non limitatif, le débit d'air aspiré par les moyens 53 d'extraction du module 22 de décontamination est de l'ordre de 600 à 800 m³ par heure.

Ainsi, cette quantité d'air est-elle négligeable par comparaison à celle qui est extraite du four 26 pour être recyclée qui est comprise entre 20.000 et 30.000 m³ par heure.

On comprendra dès lors qu'il n'est pas essentiel de recycler également cet air chargé en agent stérilisant extrait du module 22 de décontamination.

De plus, cet air n'a pas été préalablement filtré puisqu'il s'agit d'air ambiant pénétrant sans filtration dans le module 22 de décontamination par les ouvertures que comporte l'enceinte 19, de sorte que cet air ne présente pas donc le même degré de propreté que l'air filtré provenant du four 26.

De surcroît, cet air comporte une concentration plus élevée en agent stérilisant puisqu'il est extrait de la chambre 21 de stérilisation dans laquelle l'agent stérilisant est appliqué.

Ainsi, toute utilisation de cet air à d'autres fins que d'exploiter ses propriétés stérilisantes se doit de mettre en œuvre de moyens de traitement aptes à en neutraliser l'agent stérilisant et ainsi en réduire la teneur jusqu'à une valeur seuil déterminée, en particulier pour garantir l'absence de nocivité pour l'homme d'un tel air.

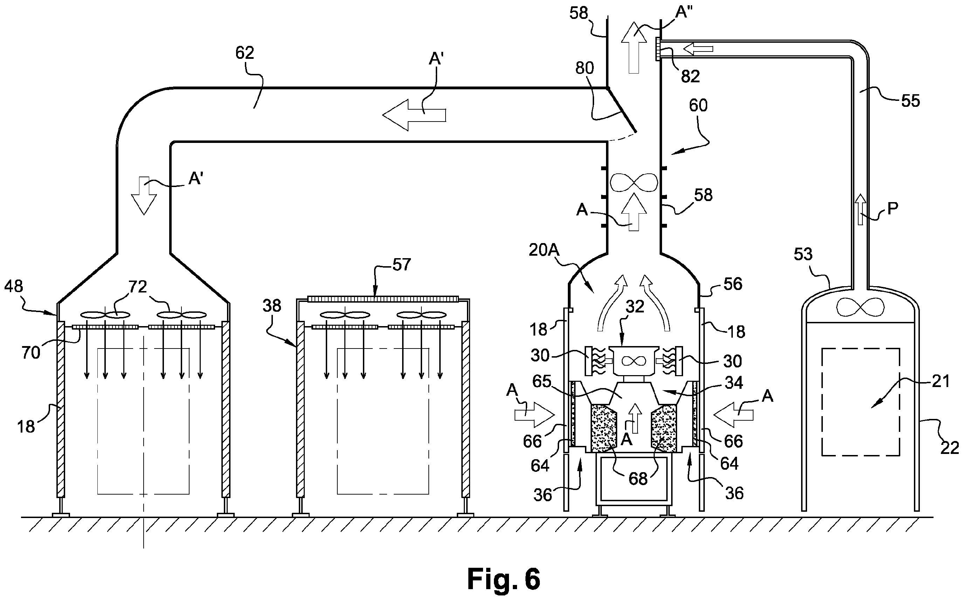

De préférence, l'air chargé en agent stérilisant provenant du module 22 de décontamination est donc évacué vers l'atmosphère, tel qu'illustré sur la figure 6 par une flèche P dans le conduit 55 d'aspiration.

De préférence, le conduit 58 d'évacuation comporte des moyens 82 de traitement de l'agent stérilisant aptes à neutraliser ledit agent de manière que la concentration finale en agent stérilisant dans l'air évacué vers l'atmosphère soit inférieure ou égale à une valeur seuil déterminée.

Avantageusement, les moyens 82 de traitement de l'agent stérilisant sont aptes à neutraliser l'agent sous forme gazeuse par une opération de catalyse.

De préférence, les moyens 82 de traitement de l'agent stérilisant sont agencés à la jonction du conduit 55 d'aspiration et du conduit 58 d'évacuation.

Lorsque les moyens 80 de régulation occupent une autre position que la première position extrême une partie de l'air filtré extrait n'est alors pas recyclée dans le conduit principal 62 et cet air évacué vers l'atmosphère permet avantageusement de réduire la concentration par dilution de l'air chargé provenant du module 22 de décontamination dans cet air filtré et extrait du four 26.

On décrira maintenant par comparaison et en référence à la figure 6, le fonctionnement d'un circuit 60 de recyclage selon le deuxième mode de réalisation de l'invention.

La circulation de l'air depuis l'aspiration de l'air ambiant dans le local industriel jusqu'à son extraction par les moyens 56 d'extraction étant identique à celle décrite précédemment en référence à la figure 4 du premier mode de réalisation, on s'y reportera avantageusement sans qu'il soit nécessaire de la répéter ici pour le deuxième mode de réalisation.

Selon l'invention, l'air de refroidissement est extrait par le haut par les moyens 56 d'extraction du circuit 60 de recyclage de l'air en vue d'être recyclé au moins dans l'unité 48 de remplissage.

L'air filtré ainsi extrait est alors recyclé par l'intermédiaire du conduit principal 62 du circuit 60 qui achemine au moins une partie de l'air jusqu'à l'unité 48 de remplissage.

De préférence, seule une partie de l'air est ici recyclée vers l'unité 48 de remplissage, le débit d'air extrait pouvant être recyclé étant supérieur au débit d'air nécessaire à l'établissement d'une surpression dans ladite unité 48 de remplissage.

Les moyens 80 de régulation dévient une partie de l'air correspondant à la flèche A" vers le conduit 58 d'évacuation pour permettre une évacuation efficace et la dilution de l'air chargé en agent stérilisant correspondant à la flèche P.

En effet, la quantité d'air filtré extraite par les moyens 56 d'extraction en vue du recyclage est généralement supérieure à celle nécessaire pour alimenter l'unité 48 de remplissage dès lors que la consommation en air de la première est par exemple de l'ordre de 20.000 à 30.000 m³ par heure quand celle la deuxième n'est que de l'ordre de 10.000 m³ par heure.

Grâce à la dilution obtenue dans le conduit 58 d'évacuation, les moyens de traitement 82 peuvent avantageusement être supprimés tout en respectant les normes environnementales relatives à la teneur en agent stérilisant de l'air évacué dans l'atmosphère.

L'air parvenu à l'unité 48 de remplissage suivant la flèche A', avantageusement avec l'assistance des moyens 72 de ventilation, est alors filtré une deuxième fois par les moyens 70 de filtration équipant l'unité 48 et introduit dans le volume intérieur 20C associé de l'enceinte 18 afin d'y maintenir en permanence une surpression autour des machines 50 et 52.

L'air s'échappe ensuite de l'unité 48 de remplissage par la partie inférieure de l'enceinte 18 pour être restitué au local industriel 16.

De préférence, l'unité 38 de transformation de l'installation 10 comporte, indépendant du circuit 60 de recyclage, un système 57 d'insufflation du type décrit précédemment dont la consommation en air de l'ordre de 8000 à 12.000 m³ par heure est représentée par une flèche B sur la figure 6.

Avantageusement, le système 57 d'insufflation comporte des moyens 76 de filtration et des moyens 78 de ventilation aptes à aspirer l'air ambiant dans le local industriel 16 pour établir une surpression dans le volume intérieur 20B de l'unité 38 de transformation des préformes 14.

## Revendications

1. Installation (10) pour la fabrication de récipients (12) à partir de préformes (14) en matière thermoplastique, comportant une enceinte (18) de protection destinée à isoler un volume intérieur (20) de l'installation (10) par rapport à l'air ambiant compris dans un local industriel (16) dans lequel est implantée l'installation (10), l'installation (10) comportant agencées dans ledit volume intérieur (20) au moins :
- une unité de conditionnement thermique des préformes (14) constituée d'un four (26) comportant des moyens (30) de chauffage et un dispositif (34) de refroidissement par air qui comporte des moyens (36) de filtration de l'air ambiant puisé dans le local industriel par des moyens (32) de ventilation pour assurer un refroidissement d'au moins une partie des préformes (14) en transit à l'intérieur du four (26) grâce audit air filtré ;
- une unité (38) de transformation des préformes (14) en récipients (12), et
- une unité de remplissage (48) des récipients (12) obtenus à partir desdites préformes (14),
**caractérisée en ce que** l'installation (10) comporte un circuit (60) de recyclage de l'air de refroidissement comportant :
- des moyens (56) d'extraction qui sont aptes à extraire hors du four (26) l'air filtré introduit à l'intérieur du four (26) par le dispositif (34) de refroidissement par air, et
- au moins un conduit (62, 74) apte à amener au moins une partie dudit air filtré extrait du four (26) jusqu'à au moins une unité (38, 48) de l'installation à l'intérieur de laquelle ledit air est introduit, grâce à quoi tout ou partie dudit air filtré initialement pour opérer un refroidissement par air dans le four (26) est recyclé au moins directement dans l'unité (38) de transformation et/ou l'unité (48) de remplissage.

2. Installation (10) selon la revendication 1, **caractérisée en ce que** le circuit (60) de recyclage comporte un conduit principal (62) dont une extrémité amont est reliée aux moyens (56) d'extraction et une extrémité aval reliée à l'unité (48) de remplissage de manière que au moins une partie de l'air filtré extrait du four (26) soit recyclée en étant introduite à l'intérieur de ladite unité (48) de remplissage.

3. Installation (10) selon l'une des revendications 1 ou 2, **caractérisée en ce que** le circuit (60) de recyclage comporte un conduit (74) de dérivation dont une extrémité amont est reliée au conduit principal (62) ou aux moyens (56) d'extraction et dont l'autre extrémité aval est reliée à l'unité (38) de transformation des préformes (14) en récipients (12) de manière que au moins une partie de l'air filtré extrait du four (26) soit recyclée en étant introduite à l'intérieur de ladite unité (38) de transformation.

4. Installation (10) selon l'une quelconque des revendications précédentes, **caractérisée en ce que** l'unité (38) de transformation et/ou l'unité (48) de remplissage comportent des moyens (76, 70) de filtration pour filtrer l'air provenant du circuit (60) de recyclage introduit dans l'une et/ou l'autre des unités (38, 48) de manière à réaliser une double filtration de l'air recyclé.

5. Installation (10) selon l'une quelconque des revendications précédentes, **caractérisée en ce que** le circuit (60) de recyclage comporte un conduit (58) d'évacuation dont une extrémité amont communique avec les moyens (56) d'extraction et dont l'autre extrémité aval débouche dans l'atmosphère, à l'extérieur du local industriel (16), et **en ce que** des moyens (80) de régulation sont disposés de manière à répartir sélectivement l'air extrait dans ledit conduit (58) d'évacuation et/ou dans ledit au moins un conduit (62, 74) du circuit (60) de recyclage d'air.

6. Installation selon la revendication 5, comportant un module (22) de décontamination des préformes (14) par application d'au moins un agent stérilisant, **caractérisée en ce que** ledit module (22) de décontamination comporte des moyens (53) d'extraction aptes à aspirer l'air comprenant de l'agent stérilisant à l'état gazeux hors d'une chambre (21) de stérilisation du module (22) de manière à évacuer hors du module (22) ledit air chargé en agent stérilisant par l'intermédiaire d'au moins un conduit (55) d'aspiration dont la sortie est reliée au conduit (58) d'évacuation débouchant dans l'atmosphère.

7. Installation selon la revendication 6, **caractérisée en ce que** le circuit (60) de recyclage comporte des moyens de traitement aptes à neutraliser tout ou partie de l'agent stérilisant présent dans l'air recyclé.

8. Installation selon la revendication 7, **caractérisée en ce que** les moyens de traitement de l'agent stérilisant sont agencés au moins dans le conduit (62, 74) apte à amener tout ou partie dudit air filtré extrait du four (26) jusqu'à au moins une unité (38, 48) de l'installation (10).

9. Installation selon la revendication 7, **caractérisée en ce que** les moyens (82) de traitement de l'agent stérilisant sont agencés dans l'un et/ou l'autre desdits conduits (55) d'aspiration et (58) d'évacuation pour neutraliser tout ou partie dudit agent stérilisant présent dans l'air.

10. Procédé de recyclage de l'air dans une installation (10) selon l'une quelconque des revendications précédentes de fabrication de récipients (12) à partir de préformes (14), comportant au moins les étapes consistant successivement à :
a) filtrer de l'air ambiant pour réaliser avec ledit air filtré un refroidissement par air d'au moins une partie des préformes (14) au cours du conditionnement thermique opéré dans un four (26) de l'installation (10) ;
b) extraire l'air filtré hors du four (26) après utilisation dudit air pour le refroidissement ;
c) recycler au moins une partie dudit air filtré extrait du four (26) en introduisant tout ou partie dudit air dans une unité (38) de transformation et/ou une unité (48) de remplissage de l'installation (10).

## Patentansprüche

1. Anlage (10) zum Herstellen von Behältern (12) aus Vorformlingen (14) aus thermoplastischem Material, die eine Schutzkammer (18) aufweist, die dazu bestimmt ist, ein Innenvolumen (20) der Anlage (10) in Bezug auf die die Umgebungsluft zu isolieren, die in einem Industriegebäude (16) enthalten ist, in dem sich die Anlage (10) befindet, wobei die Anlage (10) in dem Innenvolumen (20) mindestens Folgendes aufweist:
- eine Einheit zur thermischen Konditionierung der Vorformlinge (14), bestehend aus einem Ofen (26), der Mittel (30) zum Heizen aufweist, und einer Luftkühlvorrichtung (34), die Mittel (36) zum Filtern der Umgebungsluft aufweist, die aus dem Industriegebäude mit Mitteln (32) zum Belüften herausgezogen wird, um ein Kühlen von mindestens einem Teil der Vorformlinge (14), die sich auf dem Transport in den Ofen (26) befinden, mithilfe der gefilterten Luft sicherzustellen;
- eine Einheit (38) zum Umwandeln der Vorformlinge (14) in Behälter (12) und
- eine Einheit (48) zum Füllen der Behälter (12), die aus den Vorformlingen (14) erhalten werden,
**dadurch gekennzeichnet, dass** die Anlage (10) einen Kreis (60) zum Rückführen der Kühlluft aufweist, der Folgendes aufweist:
- Mittel (56) zum Extrahieren, die dazu geeignet sind, die in den Ofen (26) eingeführte gefilterte Luft mithilfe der Luftkühlvorrichtung (34) aus dem Ofen (26) zu extrahieren, und
- mindestens eine Leitung (62, 74), die geeignet ist, mindestens einen Teil der aus dem Ofen (26) extrahierten gefilterten Luft zu mindestens einer Einheit (38, 48) der Anlage zu leiten, in die die Luft eingeführt wird, wodurch die gesamte oder ein Teil der Luft, die anfänglich gefiltert wurde, um eine Luftkühlung in dem Ofen (26) zu betreiben, zumindest direkt in die Einheit (38) zum Umwandeln und/oder die Einheit (48) zum Füllen zurückgeführt wird.

2. Anlage (10) nach Anspruch 1, **dadurch gekennzeichnet, dass** der Kreis (60) zum Rückführen eine Hauptleitung (62) aufweist, deren vorgelagertes Ende mit den Mitteln (56) zum Extrahieren verbunden ist und deren nachgelagertes Ende mit der Einheit (48) zum Füllen so verbunden ist, dass mindestens ein Teil der aus dem Ofen (26) extrahierten gefilterten Luft zurückgeführt wird, indem sie in die Einheit (48) zum Füllen eingeführt wird.

3. Anlage (10) nach einem der Ansprüche 1 oder 2, **dadurch gekennzeichnet, dass** der Kreis (60) zum Rückführen eine Abzweigleitung (74) aufweist, deren vorgelagertes Ende mit der Hauptleitung (62) oder den Mitteln (56) zum Extrahieren verbunden ist und deren nachgelagertes Ende mit der Einheit (38) zum Umwandeln der Vorformlinge (14) in Behälter (12) so verbunden ist, dass mindestens ein Teil der aus dem Ofen (26) extrahierten gefilterten Luft zurückgeführt wird, indem sie in die Einheit (38) zum Umwandeln eingeführt wird.

4. Anlage (10) nach einem der vorausgehenden Ansprüche, **dadurch gekennzeichnet, dass** die Einheit (38) zum Umwandeln und/oder die Einheit (48) zum Füllen Mittel (76, 70) zum Filtern aufweist/aufweisen, um die Luft aus dem Kreis (60) zum Rückführen, die in die eine und/oder die andere der Einheiten (38, 48) eingeführt wird, zu filtern, um eine doppelte Filterung der zurückgeführten Luft durchzuführen.

5. Anlage (10) nach einem der vorausgehenden Ansprüche, **dadurch gekennzeichnet, dass** der Kreis (60) zum Rückführen eine Leitung (58) zum Ableiten aufweist, deren vorgelagertes Ende mit den Mitteln (56) zum Extrahieren kommuniziert und deren nachgelagertes Ende außerhalb des Industriegebäudes (16) in die Atmosphäre mündet, und dadurch, dass Mittel (80) zum Regeln so angeordnet sind, dass die extrahierte Luft wahlweise in die Leitung (58) zum Ableiten und/oder in die mindestens eine Leitung (62, 74) des Kreises (60) zum Rückführen der Luft aufgeteilt wird.

6. Anlage nach Anspruch 5, die ein Modul (22) zum Dekontaminieren der Vorformlinge (14) durch Aufbringen von mindesten einem Sterilisationsmittel aufweist, **dadurch gekennzeichnet, dass** das Modul (22) zum Dekontaminieren Mittel (53) zum Extrahieren aufweist, die geeignet sind, die Luft, die Sterilisationsmittel im gasförmigen Zustand aufweist, aus einer Kammer (21) zum Sterilisieren des Moduls (22) so anzusaugen, dass die mit Sterilisationsmittel angereicherte Luft mithilfe von mindestens einer Leitung (55) zum Absaugen, deren Ausgang mit der Leitung (58) zum Ableiten, die in die Atmosphäre mündet, verbunden ist, aus dem Modul (22) abgeleitet wird.

7. Anlage nach Anspruch 6, **dadurch gekennzeichnet, dass** der Kreis (60) zum Rückführen Mittel zum Verarbeiten aufweist, die geeignet sind, das in der rückgeführten Luft vorhandene Sterilisationsmittel ganz oder teilweise zu neutralisieren.

8. Anlage nach Anspruch 7, **dadurch gekennzeichnet, dass** die Mittel zum Verarbeiten des Sterilisationsmittels mindestens in der Leitung (62, 74) angeordnet sind, die geeignet ist, die gesamte oder einen Teil der aus dem Ofen (26) extrahierten gefilterten Luft zu mindestens einer Einheit (38, 48) der Anlage (10) zu leiten.

9. Anlage nach Anspruch 7, **dadurch gekennzeichnet, dass** die Mittel (82) zum Verarbeiten des Sterilisationsmittels in der einen und/oder der anderen der Leitungen (55) zum Absaugen bzw. Ableiten (58) angeordnet sind, um das in der Luft vorhandene Sterilisationsmittel ganz oder teilweise zu neutralisieren.

10. Verfahren zum Zurückführen von Luft in einer Anlage (10) nach einem der vorausgehenden Ansprüche zum Herstellen von Behältern (12) aus Vorformlingen (14), das mindestens die folgenden, nacheinander durchgeführten Schritte aufweist:
a) Filtern der Umgebungsluft, um mit der gefilterten Umgebungsluft eine Luftkühlung von mindestens einem Teil der Vorformlinge (14) während der thermischen Konditionierung durchzuführen, die in einem Ofen (26) der Anlage (10) erfolgt;
b) Extrahieren der gefilterten Luft aus dem Ofen (26) nach dem Verwenden der Luft zum Kühlen;
c) Rückführen mindestens eines Teils der aus dem Ofen (26) extrahierten gefilterten Luft, indem die Luft ganz oder teilweise in eine Einheit (38) zum Umwandeln und/oder eine Einheit (48) zum Füllen der Anlage (10) zurückgeführt wird.

## Claims

1. Plant (10) for manufacturing containers (12) from thermoplastic preforms (14), comprising a protective enclosure (18) intended to isolate an interior volume (20) of the plant (10) from the ambient air contained in an industrial premises (16) in which the plant (10) is installed, the plant (10) comprising, arranged in said interior volume (20), at least:
- a unit for thermally conditioning the preforms (14) which consists of an oven (26) comprising heating means (30) and an air-cooling device (34) which comprises means (36) of filtering the ambient air drawn from the industrial premises by ventilation means (32) in order to cool at least part of the preforms (14) in transit inside the oven (26) using said filtered air;
- a unit (38) for converting the preforms (14) into containers (12), and
- a unit (48) for filling the containers (12) obtained from said preforms (14),
**characterized in that** the plant (10) comprises a cooling-air recycling circuit (60) comprising:
- extraction means (56) which are able to extract from the oven (26) the filtered air introduced into the oven (26) by the air-cooling device (34), and
- at least one pipe (62, 74) able to convey at least some of said filtered air extracted from the oven (26) to at least one unit (38, 48) of the plant into which said air is introduced, by means of which all or some of said air filtered initially to perform air-cooling in the oven (26) is recycled at least directly to the conversion unit (38) and/or the filling unit (48) .

2. Plant (10) according to Claim 1, **characterized in that** the recycling circuit (60) comprises a main pipe (62) an upstream end of which is connected to the extraction means (56) and a downstream end of which is connected to the filling unit (48) so that at least some of the filtered air extracted from the oven (26) is recycled by being introduced into said filling unit (48) .

3. Plant (10) according to either of Claims 1 and 2, **characterized in that** the recycling circuit (60) comprises a branch pipe (74) an upstream end of which is connected to the main pipe (62) or to the extraction means (56) and the downstream other end of which is connected to the unit (38) that converts the preforms (14) into containers (12) so that at least some of the filtered air extracted from the oven (26) is recycled by being introduced into said conversion unit (38).

4. Plant (10) according to any one of the preceding claims, **characterized in that** the conversion unit (38) and/or the filling unit (48) comprise filtration means (76, 70) for filtering the air from the recycling circuit (60) that is introduced into one and/or the other of the units (38, 48) so as to perform double filtration on the recycled air.

5. Plant (10) according to any one of the preceding claims, **characterized in that** the recycling circuit (60) comprises a discharge pipe (58), an upstream end of which communicates with the extraction means (56) and the downstream other end of which opens into the atmosphere, outside the industrial premises (16), and **in that** regulating means (80) are arranged so as selectively to distribute the extracted air into said discharge pipe (58) and/or into said at least one pipe (62, 74) of the air recycling circuit (60).

6. Plant according to Claim 5, comprising a module (22) for decontaminating the preforms (14) by applying at least one sterilizing agent, **characterized in that** said decontamination module (22) comprises extraction means (53) able to suck up the air containing the sterilizing agent in the gaseous state from a sterilization chamber (21) of the module (22) so as to discharge from the module (22) said air laden with sterilizing agent via at least one suction pipe (55) the outlet of which is connected to the discharge pipe (58) that opens into the atmosphere.

7. Plant according to Claim 6, **characterized in that** the recycling circuit (60) comprises treatment means able to neutralize all or some of the sterilizing agent present in the recycled air.

8. Plant according to Claim 7, **characterized in that** the sterilizing-agent treatment means are arranged at least in the pipe (62, 74) able to convey all or some of said filtered air extracted from the oven (26) to at least one unit (38, 48) of the plant (10).

9. Plant according to Claim 7, **characterized in that** the sterilizing-agent treatment means (82) are arranged in one and/or the other of said suction pipe (55) and said discharge pipe (58) in order to neutralize all or some of said sterilizing agent present in the air.

10. Method for recycling air in a plant (10) according to any one of the preceding claims for manufacturing containers (12) from preforms (14), comprising at least the steps which consist in successively:
a) filtering ambient air in order, using said filtered air, to air-cool at least part of the preforms (14) while they are undergoing thermal conditioning in an oven (26) of the plant (10);
b) extracting the filtered air from the oven (26) after said air has been used for cooling;
c) recycling at least some of said filtered air extracted from the oven (26) by introducing all or some of said air into a conversion unit (38) and/or a filling unit (48) of the plant (10).
